# EUROPÄISCHE PATENTANMELDUNG

(11) **EP 0 714 027 A2**
(43) Veröffentlichungstag der Anmeldung: **29.05.1996**
(21) Anmeldenummer: 95117192.5
(22) Anmeldetag: 01.11.1995
(51) Int. Cl.: G01N 33/483, A61K 41/00, G06F 19/00, G01N 35/00, G01N 37/00, G11B 20/10, G01R 23/16, A61N 1/00

(54) **Vorrichtung und Verfahren zur Aufzeichnung von substanzspecifizischen und körperspezifischen energetischen Informationen**

(30) Priorität: 23.11.1994 AT 446/94
(71) Anmelder: 5E Systeme für holistische Medizin Ges.m.b.H., A-3100 St. Pölten (AT)
(72) Erfinder: Dillinger, Klaus, Dipl.-Ing., A-3100 St. Pölten (AT); Steiner, Christian, Dr., A-9073 Viktring/Kärnten (AT)
(74) Vertreter: Grabherr-Puchberger, Claudia

(57) **Zusammenfassung**

Mit einer Vorrichtung zur Aufzeichnung von substanzspezifischen energetischen Informationen in Form von elektromagnetischen Spektren wird nach einem erfindungsgemäßen Verfahren über ein Kontaktelement (2) ein Spektrum aufgenommen, das von einem Verstärker (3) verstärkt, von einem A/D-Konverter (7) digitalisiert und schließlich von einer Datenverarbeitungsanlage (8) auf einem elektronischen Speichermedium (9) abgespeichert wird. Erfindungsgemäß können derart abgespeicherte Spektren in jeder beliebigen Kombination über eine Datenverarbeitungsanlage (16, 28) und einen D/A-Konverter in ein analoges Signal gewandelt werden, durch einen Verstärker verstärkt und dann durch mindestens eine Elektrode abgestrahlt werden. Bei einer Vorrichtung zur Wiedergabe von körperspezifischen, energetischen Informationen aus einem analogen Speicher werden nach einem erfindungsgemäßen Verfahren die ausgelesenen und verstärkten Spektren einem oder mehreren Bankpaßfiltern zugeführt, deren Ausgangssignale in einer logischen Einheit beliebig kombiniert werden, wobei die Frequenzbereiche der Bandpaßfilter homöopathischen Potenzen entsprechen.

## Beschreibung

Die Erfindung betrifft eine Vorrichtung und ein Verfahren zur Aufzeichnung von substanz- und körperspezifischen energetischen Informationen in Form von elektromagnetischen Spektren. Die Erfindung betrifft weiters Vorrichtungen und Verfahren zur Wiedergabe von substanz- und körperspezifischen energetischen Informationen in Form von elektromagnetischen Spektren, insbesondere eine Vorrichtung und ein Verfahren zur Erzeugung von therapeutischer Strahlung sowie eine Vorrichtung und ein Verfahren zur Erzeugung von energetischen Medikamenten.

Jede chemische Substanz sendet bei Zimmertemperatur ein elektromagnetisches Rauschen aus, welches für die jeweilige Substanz charakteristische Information enthält. Auf dem Prinzip dieser substanzspezifischen ultrafeinen Schwingungsinformationen beruhen ganzheitliche Therapieverfahren, wie unter anderem die Homöopathie, bei welcher sich die Substanzinformation durch die Verschüttelungsvorgänge beim Potenzieren auf Wassercluster aufprägt. Derartig hergestellte Homöopathika sind sehr sensibel in bezug auf die Lagerung, da Einflüsse von elektrischen oder magnetischen Feldern in der Umgebung des Lagerplatzes, die in den Homöopthika enthaltene Information stören können und die Wirkung des Homöopathikums damit herabsetzen. Ebenfalls auf dem Prinzip ultrafeiner Schwingungsinformation beruht die Bioresonanz, wo bestimmte Transformationen der Substanzschwingung zum Testen und Löschen von Allergien Verwendung finden. Die Bioresonanz erfaßt diagnostisch und therapeutisch die Körpersysteme der Organebene des Bindegewebes und des Vegetativums. Nicht erfaßt werden von der Bioresonanz die Körpersysteme der folgenden Ebenen.
- Die Ebene der bewußten Muskelsteuerung (Kleinhirn, Sulcus Rolandi mit dem Körperfühl- und Bewegungszentrum und die übergeordneten Rindenzentren zur Umsetzung von Absichten). Dieser Ebene assoziiert sind auch die Triebe (die ja ebenfalls primitiven Absichten entsprechen.
- Die Ebene der Sinnesorgane (Auge, Ohr, Geschmack..) + deren Verarbeitungszentren im Gehirn (Sehrinde, Hörrinde, etc.), aber auch alle Zentren, die den unmittelbaren Sinneseindruck auswerten und zur (Lebens-)Erfahrung verdichten.
- Die Ebene des abstrakten Denkens, das unabhängig von Körper- oder Sinnesreizen funktioniert und die höheren Funktionen des Egos wiederspiegelt. Die entsprechenden Hirnzentren sind nach Seitelberger und Eccles wahrscheinlich hologrammartig über die ganze Großhirnrinde verteilt.

Im Sinne der ganzheitlichen Medizin ist jedoch die Erfassung dieser drei Ebenen bei der Diagnose und der Therapie von großer Bedeutung.

Aufgabe der vorliegenden Erfindung ist es, Vorrichtungen und Verfahren zu finden, die die Aufzeichnung und Wiedergabe von substanz- und körperspezifischen energetischen Informationen in Form von elektromagnetischen Spektren ermöglichen, ohne die obengenannten Nachteile mit sich zu bringen. Insbesondere soll auch eine Vorrichtung und ein Verfahren zur Erzeugung von energetischen Medikamenten geschaffen werden, die bzw. das eine ständige Verfügbarkeit von qualitativ hochwertigen homöopathischen Medikamenten beliebiger Zusammensetzung ermöglicht.

Ferner sollen Vorrichtungen zur Therapie und zur Diagnose geschaffen werden, die jeden der sechs grundlegenden Bereiche, Organe/Bindegewebe, Stütz- und Bewegungsapparat/Vegetativum/bewußte Muskelsteuerung und Triebe (=Selbstverwirklichung)/Sinnesorgane und Auswertung (=Lebenserfahrung)/abstraktes Denken (=Ego), getrennt erfaßbar macht.

Die gestellten Aufgaben werden erfindungsgemäß durch eine Vorrichtung zur Aufzeichnung erfüllt, die ein ein Spektrum aufnehmendes Kontaktelement, das mit eine Verstärker verbunden ist, einen A/D-Konverter, der das verstärkte Signal digitalisiert und eine Datenverarbeitungsanlage, die das digitalisierte Signal auf einem elektronischen Speichermedium speichert, enthält. Vorzugsweise enthält die Vorrichtung einzeln ansteuerbare Bandpaßfilter, deren Frequenzbereiche homöopathischen Potenzen bzw. den folgenden grundlegenden Bereichen - Organe; Bindegewebe, Stütz- und Bewegungsapparat; Vegetativum; bewußte Muskelsteuerung und Triebe (i.e. Selbstverwirklichung); Sinnesorgane und Auswertung (i.e. Lebenserfahrung); abstraktes Denken (i.e.Ego) entsprechen.

Vorteilhafterweise sind sechs wahlweise ansteuerbare Bandpaßfilter vorgesehen, deren Frequenzbereiche den homöopathischen Potenzen D3, D4, D12, D21, D30 und D200 entsprechen.

Gemäß dem erfindungsgemäßen Verfahren zur Aufzeichnung von substanzspezifischen und energetischen Informationen in Form von elektromagnetischen Spektren wird über ein Kontaktelement ein Spektrum aufgenommen, das von eine Verstärker verstärkt, dann von eine A/D-Konverter digitalisiert und schließlich von einer Datenverarbeitungsanlage auf einem elektronischen Speichermedium abgespeichert wird. Vorteilhafterweise wird das Signal wahlweise einzelansteuerbaren Bandpaßfiltern mit verschiedenen homöopathischen Potenzen zugeordneten Frequenzbereichen zugeführt, und die so erhaltenen für homöpathische Potenzen charakteristischen Signale werden getrennt abgespeichert.

Das digitalisierte Signal wird erfindungsgemäß vor dem Abspeichern von der Datenverarbeitungsanlage einer Fehlerkompensation zur Korrektur der Signalverfälschung durch die von der Datenverarbeitunganlage erzeugte elektromagnetische Eigenschwingung unterworfen.

Die erfindungsgemäße Vorrichtung und das erfindungsgemäße Verfahren zur Aufzeichnung von substanzspezifischen energetischen Informationen können zur praktisch unbegrenzten Aufbewahrung von energetischen Informationen verwendet werden.

Die erfindungsgemäße Vorrichtung zur Wiedergabe von substanzspezifischen energetischen Informationen in Form von elektromagnetischen Spektren enthält einen elektronischen Speicher mit darin abgespeicherten Spektren, eine Datenverarbeitungsanlage, einen D/A-Konverter.

Gemäß dem erfindungsgemäßen Verfahren zur Wiedergabe werden ein oder mehrere ausgewählte substanzspezifische Spektren (Spektrum) aus einer Vielzahl auf einem Speichermedium gespeicherten Spektren in eine Datenverarbeitungsanlage geladen, über einen D/A-Konverter in ein analoges Signal gewandelt, durch einen Verstärker verstärkt und dann durch mindestens eine Elektrode abgestrahlt.

Die Vorrichtung und das Verfahren zur Wiedergabe können zur Erzeugung von therapeutischen Strahlen aber auch zur Herstellung von energetischen Medikamenten verwendet werden. Die Datenverarbeitungsanlage ermöglicht die beliebige Verarbeitung des digitalisierten Spektrums, wie die Invertierung, das Aufsummieren mehrerer Spektren, das Gewichten der einzelnen Spektren vor deren Aufsummierung.

Die erfindungsgemäßen Vorrichtungen und Verfahren führen zu einer Art "digitalen" Homöopathie, indem sie z.B. die niederfrequenten Anteile des Schwingungsspektrums digitalisieren, in eine Datenverarbeitungsanlage speichern, verarbeiten und analogisiert wiedergeben können.

Diese wiedergegebenen Substanzschwingungen können in allen Disziplinen der energetischen Medizin für Diagnose und Therapie eingesetzt werden. Durch entsprechende Messungen am Patienten (z.B. mittels Elektroakupunktur nach Voll) kann man zeigen, daß sich die energetischen Auswirkungen von digital gesampelten Substanzschwingungen auf den menschlichen Körper völlig äquivalent zu echten Substanzproben bzw. echten Homöopathika verhalten und sogar eine bessere Qualität als entsprechende analoge Kopien aufweisen. Das letztere Phänomen wird darauf zurückgeführt, daß analoge Substanzproben durch unsachgemäße Lagerung (Einflüsse von elektrischen und magnetischen Feldern) in ihrer spezifischen Wirksamkeit weentlich beeinträchtigt werden können, während digitale Substanzproben naturgemäß keinem Alterungsprozeß unterliegen und somit immer in gleichbleibender Qualität und Reinheit bereitstehen.

In digitalisierter Form können die Substanzschwingungen zudem beliebig durch Computerprogramme verarbeitet und damit Systeme mit bisher unerreichter diagnostischer und therapeutischer Effizienz bei gleichzeitig maximalem Bedienungskomfort implementiert werden. Insbesondere ermöglicht die digitalisierte Form der Substanzschwingungen die
- Verwaltung von mehreren tausend digitalisierten Substanzproben auf einer CD-ROM,
- die softwaremäßige homöopathische Potenzierung von Substanzen,
- das softwaremäßige Mischen von beliebig vielen digitalisierten Substanzproben in unterschiedlichen Potenzen in beliebigen Mischungsverhältnissen (= Erstellung eines virtuellen homöopathischen Komplexes),
- die softwaremäßige Inversion von digitalisierten Substanzproben (entspricht Inversion der Substanzschwingungen z.B.zur Allergielöschung),
- die computergestützte automatisierte Allergiesuche,
- die computergestützte Therapie durch Aufschwingen von transformierten Substanzschwingungen auf den Patienten (z.B. Allergielöschung mittels Bioresonanz),
- die computergestützte Rezeptierung und Herstellung von energetischen Medikamenten ("Allergietropfen") durch Aufschwingen der gespeicherten bzw. errechneten Substanzinformationen auf ein Alkohol/Wasser-Gemisch.

Die erfindungsgemäße Vorrichtung zur Wiedergabe von körperspezifischen energetischen Informationen in Form von elektromagnetischen Schwingungen enthält einen Analogspeicher mit darin abgespeicherten körperspezifischen Spektren und einen Verstärker, wobei dem Verstärker getrennt ansteuerbare Bandpaßfilter nachgeschaltet sind, deren Ausgangssignale mittels einer logischen Einheit beliebig kombinierbar sind, wobei die Frequenzbereiche der Bandpaßfilter homöopathischen Potenzen bzw. den folgenden grundlegenden Bereichen - Organe; Bindegewebe, Stütz- und Bewegungsapparat; Vegetativum; bewußte Muskelsteuerung und Triebe (i.e. Selbstverwirklichung); Sinnesorgane und Auswertung (i.e. Lebenserfahrung); abstraktes Denken (i.e.Ego) entsprechen. Die Frequenzbereiche entsprechen vorzugsweise den homöopathischen Potenzen D3, D4, D12, D21, D30 und D200. Weiters enthält die Vorrichtung vorzugsweise einen Generator zur Erzeugung einer Trägerwelle. Bei Verwendung der Vorrichtung als Therapiegerät ist diese zur Abgabe der Schwingungen mit zwei Magnetköpfen und gegebenenfalls einer Reizstrom-Handelektrode versehen, durch die patienteneigene Schwingungen rückgekoppelt werden.

Erfindungsgemäß sind die körperspezifischen Spektren in dem Kristallgitter eines Festkörpers gespeichert. Vorzugsweise werden als körperspezifische Spektren die Spektren gesunder Personen verwendet.

Bei dem erfindungsgemäßen Verfahren zur Wiedergabe von körperspezifischen energetischen Informationen in Form von elektromagnetischen Schwingungen werden diese aus einem analogen Speicher mit darin abgespeicherten körperspezifischen Spektren ausgelesen und einem Verstärker zugeleitet, wobei das aus dem Verstärker austretende Signal einem oder mehreren Bandpaßfiltern zugeführt wird. Die Ausgangssignale der Bandpaßfilter werden in einer logischen Einheit beliebig kombiniert und das kombinierte Signal wird einer oder mehreren Elektroden zugeleitet. Die Frequenzbereiche der Bandpaßfilter entsprechen dabei homöopathischen Potenzen bzw. den folgenden grundlegenden Bereichen - Organe; Bindegewebe, Stütz- und Bewegungsapparat; Vegetativum; bewußte Muskelsteuerung und Triebe (i.e. Selbstverwirklichung); Sinnesorgane und Auswertung (i.e. Lebenserfahrung); abstraktes Denken (i.e.Ego). Die Frequenzbereiche entsprechen vorzugsweise den homöopatischen Potenzen D3, D4, D12, D21, D30 und D200. Das verstärkte Signal wird zur Erzeugung einer biologisch wirkenden Schwingung durch eine der Schuhmannwelle ähnlichen Trägerwelle überlagert, die vorzugsweise aus einem 10Hz-Magnetfeld mit 1 MHz-Impulsen zusammengesetzt wird. Vorzugsweise werden durch Überlagerung des körperspezifischen Spektrums mit patienteneigenen Schwingungen Resonanzzustände erzeugt.

Die erfindungsgemäßen Vorrichtungen und Verfahren ermöglichen die diagnostische und therapeutische Erfassung aller Körpersysteme, indem jedem der sechs grundlegenden Bereiche, Organe/Bindegewebe, Stütz- und Bewegungsapparat/Vegetativum/bewußte Muskelsteuerung und Triebe (= Selbstverwirklichung)/Sinnesorgane und Auswertung (=Lebenserfahrung)/abstraktes Denken (= Ego), ein bestimmtes Frequenzband zugeordnet werden kann, innerhalb dessen homöopathische Signale für die jeweilige Ebene wirksam werden. Umgekehrt kann aus dem quasi unbegrenzten Frequenzspektrum des Körpers durch das Anlegen von Filtern entsprechend den genannten Frequenzbändern eine spezifische Information über die einzelnen Ebenen gewonnen werden, die, wie in der bisherigen Bioresonanz, therapeutisch genutzt werden. Dabei werden klassische Akupunkturmeridiane mittels elektronisch veränderter, z.B. invertierter Potenzialschwingungen anderer Punkte oder von Hand oder Fuß des Patienten aktiviert. Ebenso können z.B. die Infrarotschwingungen von Substanzen, z.B.von Körpersekreten, elektronisch erfaßt und in bestimmten Frequenzbereichen oder in invertierter Form dem Patienten übertragen werden, wodurch homöopathische Effekte auftreten, z.B. die Löschung von Allergien.

In Folge wird die Erfindung anhand der beiliegenden Zeichnungen, die Vorrichtungen in schematischer Weise darstellen, näher erklärt. Die Fig. 1 zeigt eine Vorrichtung zum Aufnehmen von substanzspezifischen Informationen. Die Fig. 2 zeigt eine Vorrichtung zur Wiedergabe von substanzspezifischen Informationen. Die Fig. 3 zeigt eine Vorrichtung zur Therapie. Die Fig. 4 stellt die Wellenform eines Trägermagnetfeldes dar.

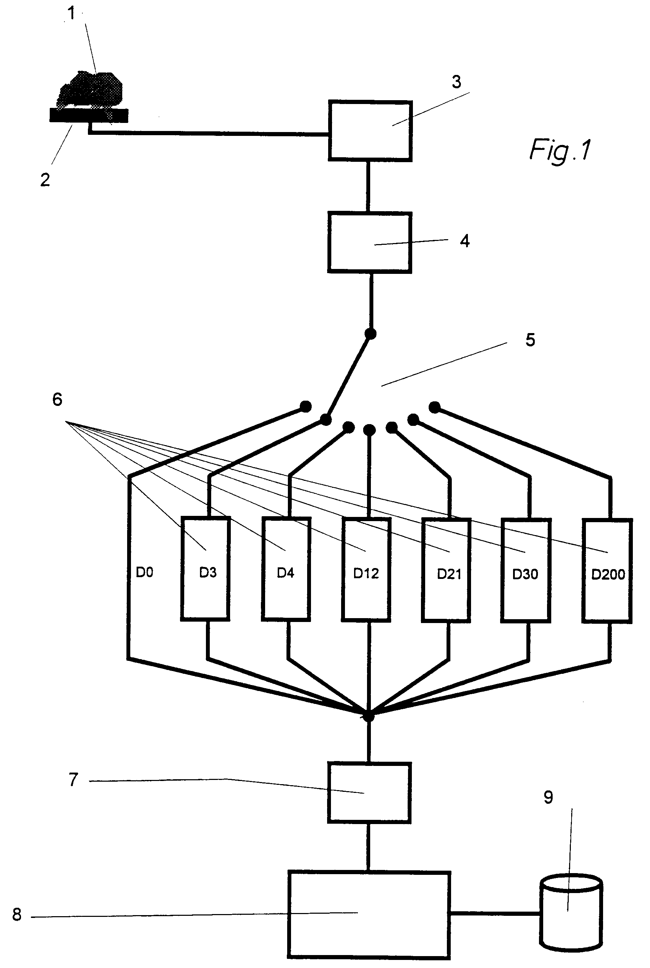

Die Vorrichtung für die Aufnahme von Substanzproben besteht aus folgenden Komponenten (Fig. 1):
- Die aufzunehmende Substanzprobe 1 wird auf eine Messingplatte 2 aufgebracht, welche als Kontaktmedium für das substanzspezifische langwellige elektromagnetische Rauschen dient.
- Damit verbunden ist ein Verstärker 3, der die von der Messingplatte ankommenden Rauschsignale elektronisch verstärkt.
- Anschließend wird das verstärkte Rauschsignal über einen Tiefpaßfilter 4 und einen manuellen Schalter 5 zu einem von sechs Bandpaßfiltern 6 geleitet. Der Schalter wird vom Bediener entsprechend der gewünschten aufzunehmenden homöopathischen Potenz eingestellt. Zwischen den einzelnen homöopathischen Potenzen und den Filterfrequenzen besteht folgender Zusammenhang:

| **POTENZ** | **FILTERFREQUENZEN** |
|---|---|
| DO (Ursubstanz) | 0 - 22 KHz |
| D3 | 0 - 3,5 KHz |
| D4 | 3,5 - 7 KHz |
| D12 | 7 - 10,5 KHz |
| D21 | 10,5 - 14 KHz |
| D30 | 14 - 18 KHz |
| D200 | 18 - 22 KHz |

- Nach dem Durchlauf durch den Bandpaßfilter wird das Signal in einem Analog/Digitalkonverter 7 mit einer Samplingrate von 44,1 KHz bei einer Auflösung von 16 Bit eine Sekunde lang digital gesampelt.
- Die nunmehr digitalen Schwingungsdaten werden in einen Computer 8 übertragen, wo sie mit Hilfe eines speziellen Softwareprogrammes einem Fehlerkompensationsalgorithmus unterzogen werden. Diese Fehlerkompensation ist erforderlich, um die bei Aufnahme und Wiedergabe durch die elektromagnetische Eigenschwingung des Computersystems entstehende Signalverfälschung zu korrigieren.
- Anschließend werden die fehlerbereinigten Schwingungsdaten in einer Datenbank registriert und auf ein optisches Speichermedium 9, z.B. CD-ROM, aufgebracht.
- Damit ist der Aufnahmeprozeß für eine Substanz abgeschlossen.

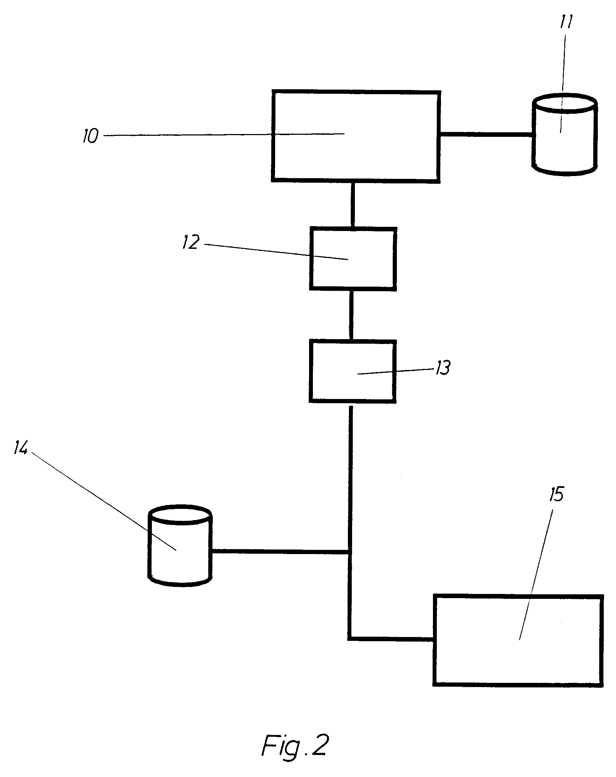

Die Vorrichtung für die Wiedergabe von Substanzproben besteht aus folgenden Komponenten (Fig. 2):
- Im Computer 10, welcher eine Datenbank mit den digital gespeicherten Substanzproben 11 enthält, läuft ein speziell entwickeltes Softwareprogramm. Mit Hilfe dieses Programmes wählt der Anwender die zu mischenden Substanzen und die anzuwendende homöopathische Potenzierung aus. Das Programm liest die benötigen Substanzproben in der benötigten Potenz aus der Datenbank und addiert die Schwingungen der zu mischenden Substanzen.
- Die resultierenden Daten werden an einen Digital/Analog-Konverter 12 übertragen, welcher die Umsetzung der digitalen in analoge elektromagnetische Schwingungen vornimmt. Da die Dauer des ursprünglich digitalisierten Signals nur 1 sek beträgt, wird diese Wiedergabe vom Computerprogram in einer Schleife endlos wiederholt, um ein kontinuierliches Ausgabesignal zu erhalten. Dieses enthält nun die gemischten feinenergetischen substanzspezifischen Informationen der ursprünglichen Substanzproben.
- Das analoge Signal wird nun über einen Verstärker 13 zu einen Therapiegerät 15 geleitet. Dieses schwingt die Substanzinformationen mittels eines speziellen Verfahrens auf den Patienten auf.
- Alternativ dazu kann die Substanzinformation auch mittels einer Becherelektrode 14 auf ein Alkohol-Wasser-Gemisch aufgeschwungen und auf diese Weise ein energetisches Medikament hergestellt werden.

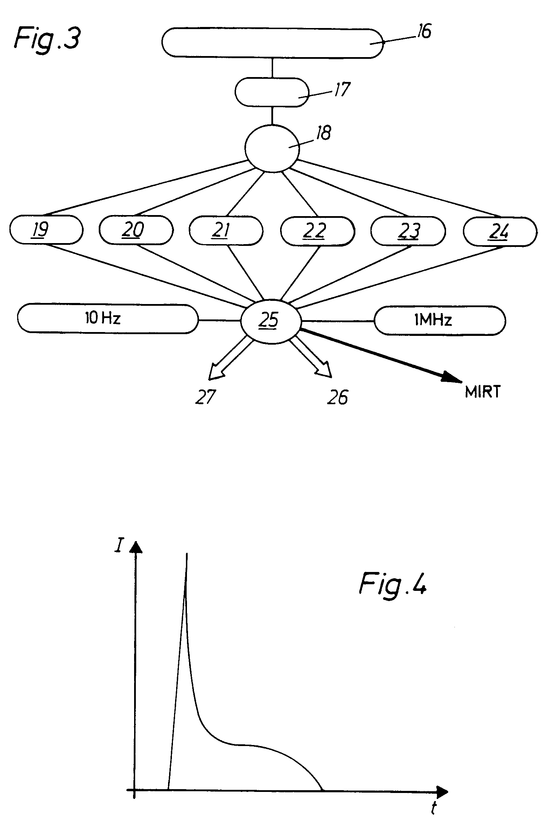

Die Vorrichtung gemäß Fig. 3 enthält einen analogen Speicher 16, in dem die Potentialschwingungen von wesentlichen Akupunkturpunkten normaler Versuchspersonen gespeichert werden und bei Bedarf ausgelesen werden können. Das entsprechende Spektrum wird einem Verstärker 17 und danach einer Logik 18 zugeführt, die Bandpaßfilter 19 bis 24 einzeln ansteuern kann. Die Ausgangssignale der Bandpaßfilter können von einer weiteren Logik 25 zur Erzeugung eines therapeutischen Magnetfeldes, das über Magnetköpfe 26, 27 abgegeben wird oder zur minimalen Reizstromtherapie MIRT verwendet werden.

Dabei werden die eigentlichen Schwingungsinformationen nach dem Prozessing durch die Bandpaßfilter einem oszillierenden 10 Hz Magnetfeld und einem 1 MHz Impuls überlagert. Diese Trägerwelle ist in Fig. 4 dargestellt. Beide Wellenformen fungieren dabei als Träger der o.a. Schwingungsinformationen, die erst deren Bioverfügbarkeit ermöglichen, da sie tief ins Gewebe eindringen und dort Resonanzphänomene hervorrufen. Der Effekt wird verstärkt durch eine minimale Reizstromtherapie, wobei der angelegte Schwachstrom (3V) gleichartig wie das Magnetfeld gepulst ist und wiederum als Träger der genannten Schwingungsinformationen fungiert (diesmal auf galvanischer Basis).

Was das Trägetmagnetfeld betrifft, so entspricht der Kurvenverlauf und auch die Frequenz des anliegenden Magnetfeldes weitgehend den Schuhmann-Wellen, die sich als stehende Wellen der Magnetosphäre zwischen Erdoberfläche und Magnetosphäre aufbauen.

Diese Wellenform ist als Träger biologisch relevanter Informationen optimal geeignet, da sich der Mensch in entwicklungsgeschichtlichen Zeiträumen maximal an sie adaptiert hat und daher mit ihr maximal in Resonanz treten kann.

Sowohl in der Diagnose (nach dem Muster der Elektroakupunktur) als auch in der Therapie (als Magnetfeldtherapie, kombiniert mit einer minimalen Reizstromtherapie) erfolgt die klare Zuordnung der ursprünglich gespeicherten, dem Magnetfeld und dem Reizstrom überlagerten Information zu den einzelnen Ebenen über die Bandpaßfilter, die je einer Ebene entsprechen.

Mittels Feinabstimmung der erfindungsgemäßen Vorrichtung (Phasenverschiebung 10 Hz-Signals an den Magnetköpfen, unterschiedliche Zumischung eines zusätzlichen HF-Signals) läßt sich auch innerhalb einer Ebene eine Zuordnung beispielsweise zu einzelnen Organen (Ebene 1) oder zu Unterfunktionen des Bindegewebes, sowie des Stütz- und Bewegungsapparates (Ebene 2) sowie auch zu Detailfunktionen des Zentralnervensystemes (Ebene 4-6) vornehmen.

Ebenso können während der Therapie die patienteneigenen Schwingungen, die in Echtzeit (z.B. von der hand oder von ausgewählten Akupunkturpunkten) angegriffen werden, nach dem gleichen Muster prozessiert und wieder zurückgespielt werden, wodurch therapeutische Resonanzphänomene auftreten.

Durch die klare Zuordnung sowohl der gespeicherten Referenzschwingung als auch der prozessierten Patientenschwingung zu den sech hierarchischen Haupt- und auch den darin enthaltenen Subsystemen geht das Gerät über den bisher üblichen diagnostischen und therapeutischen Rahmen der Bioresonanz weit hinaus.

## Patentansprüche

1. Vorrichtung zur Aufzeichnung von substanzspezifischen energetischen Informationen in Form von elektromagnetischen Spektren, dadurch gekennzeichnet, daß die Vorrichtung ein ein Spektrum aufnehmendes Kontaktelement (2), das mit einem Verstärker (3) verbunden ist, einen A/D-Konverter (7), der das verstärkte Signal digitalisiert, und eine Datenverarbeitungsanlage (8), die das digitalisierte Signal auf einem elektronischen Speichermedium (9) speichert, enthält.

2. Vorrichtung nach Anspruch 1, dadurch gekennzeichnet, daß die Vorrichtung weiters einzeln ansteuerbare Bandpaßfilter (6) enthält, deren Frequenzbereiche homöopathischen Potenzen bzw. den folgenden grundlegenden Bereichen - Organe; Bindegewebe, Stütze- und Bewegungsapparat; Vegetativum; bewußte Muskelsteuerung und Triebe (i.e. Selbstverwirklichung); Sinnesorgane und Auswertung (i.e. Lebenserfahrung); abstraktes Denken (i.e.Ego) entsprechen.

3. Vorrichtung nach Anspruch 2, dadurch gekennzeichnet, daß sechs wahlweise ansteuerbare Bandpaßfilter (6) vorgesehen sind, deren Frequenzbereiche den homöopathischen Potenzen D3, D4, D12, D21, D30 und D200 entsprechen.

4. Verfahren zur Aufzeichnung von substanzspezifischen energetischen Informationen in Form von elektromagnetischen Spektren, dadurch gekennzeichnet, daß über ein Kontaktelement (2) ein Spektrum aufgenommen wird, das von einem Verstärker (3) verstärkt, dann von einem A/D-Konverter (7) digitalisiert und schließlich von einer Datenverarbeitungsanlage (8) auf einem elektronischen Speichermedium (9) abgespeichert wird.

5. Verfahren nach Anspruch 4, dadurch gekennzeichnet, daß das aus dem Verstärker austretende Signal wahlweise einzeln ansteuerbaren analogen Bandpaßfiltern (6) bzw. das aus dem A/D-Konverter (7) austretende Signal entsprechenden digitalen Bandpaßfiltern mit verschiedenen, homöopathischen Potenzen zuordenbaren Frequenzbereichen zugeführt wird und die so erhaltenen für die homöopathischen Potenzen charakteristischen Signale getrennt gespeichert werden.

6. Verfahren nach Anspruch 5, dadurch gekennzeichnet, daß das Signal wahlweise sechs Bandpaßfilter (6) zugeleitete werden kann, deren Frequenzbereiche den homöopathischen Potenzen D3, D4, D12, D21, D30 und D200 entsprechen.

7. Verfahren nach einem der vorhergehenden Ansprüche 4 bis 6, dadurch gekennzeichnet, daß das digitalisierte Signal vor dem Abspeichern von der Datenverarbeitungsanlage (8) einer Fehlerkompensation zur Korrektur der Signalverfälschung durch die von der Datenverarbeitunganlage (8) erzeugte elektromagnetische Eigenschwingung unterworfen wird.

8. Vorrichtung zur Wiedergabe von substanzspezifischen energetischen Informationen in Form von elektromagnetischen Schwingungen, dadurch gekennzeichnet, daß die Vorrichtung einen elektronischen Speicher (11, 16, 28) mit darin abgespeicherten substanzspezifischen Spektren, eine Datenverarbeitungsanlage (18) und einen D/A-Konverter enthält.

9. Vorrichtung nach Anspruch 8, dadurch gekennzeichnet, daß die Vorrichtung zur Erzeugung therapeutischer, elektromagnetischer Schwingungen verwendet wird.

10. Vorrichtung nach Anspruch 8, dadurch gekennzeichnet, daß sie zusätzlich eine ein Trägermedium enthaltende Becherelektrode enthält und die Vorrichtung zur Erzeugung von energetischen Medikamenten verwendet wird.

11. Verfahren zur Wiedergabe von substanzspezifischen energetischen Informationen in Form von elektromagnetischen Schwingungen, dadurch gekennzeichnet, daß ein oder mehrere ausgewählte(s) substanzspezifische(s) Spektren (Spektrum) aus einer Vielzahl von auf einem Speichermedium gespeicherten Spektren in eine Datenverarbeitungsanlage (16, 28) geladen über einen D/A-Konverter in ein analoges Signal gewandelt, durch einen Verstärker (17) verstärkt und dann durch mindestens eine Elektrode (26, 27) abgestrahlt werden(wird).

12. Verfahren nach Anspruch 11, dadurch gekennzeichnet, daß dieses Verfahren zur Erzeugung von therapeutischen, elektromagnetischen Schwingungen verwendet wird, die über die Elektroden eines Therapiegerätes auf den Patienten aufgeschwungen werden.

13. Verfahren nach einem der Ansprüche 11 oder 12, dadurch gekennzeichnet, daß die in die Datenverarbeitungsanlage geladenen Spektren nach ihrer homöopathischen Potenz getrennt abgespeichert sind.

14. Verfahren nach einem der Ansprüche 11 bis 13, dadurch gekennzeichnet, daß das oder die eingelesenen Spektren vor der Analogisierung invertiert, aufsummiert oder gewichtet und aufsummiert werden und zur Erzeugung einer über eine bestimmte Zeit andauernden Strahlung entsprechend oft wiederholt werden.

15. Verfahren nach einem der Ansprüche 11, 13 oder 14, dadurch gekennzeichnet, daß die durch das Verfahren erzeugte Schwingung und damit die energetische Information einer oder mehrerer Substanz(en) bzw. die energetische Information einer oder mehrerer homoöpathischer Potenzen der Substanz(en) oder eine Summe aus den genannten Informationen in beliebiger Gewichtung über eine Becherelektrode auf ein darin befindliches Trägermedium übertragen wird und somit ein energetisches Medikament erzeugt wird.

16. Vorrichtung zur Wiedergabe von körperspezifischen energetischen Informationen in Form von elektromagnetischen Schwingungen, die einen analogen Speicher (11, 16, 28) mit darin abgespeicherten körperspezifischen Spektren und einen Verstärker enthält, dadurch gekennzeichnet, daß dem Verstärker getrennt ansteuerbare Bandpaßfilter (19 bis 24) nachgeschaltet sind, deren Ausgangssignale mittels einer logischen Einheit (25) beliebig kombinierbar sind, wobei die Frequenzbereiche der Bandpaßfilter homöopathischen Potenzen bzw. den folgenden grundlegenden Bereichen - Organe; Bindegewebe, Stütz- und Bewegungsapparat; Vegetativum; bewußte Muskelsteuerung und Triebe (i.e. Selbstverwirklichung); Sinnesorgane und Auswertung (i.e. Lebenserfahrung); abstraktes Denken (i.e.Ego) entsprechen.

17. Vorrichtung nach Anspruch 16, dadurch gekennzeichnet, daß sechs wahlweise ansteuerbare Bandpaßfilter (6) vorgesehen sind, deren Frequenzbereiche den homöopathischen Potenzen D3, D4, D12, D21, D30 und D200 entsprechen.

18. Vorrichtung nach Anspruch 16 oder 17, dadurch gekennzeichnet, daß mindestens ein Generator zur Erzeugung einer Trägerwelle mit der logischen Einheit (25) verbunden ist.

19. Vorrichtung nach Anspruch 16, 17 oder 18 zur Verwendung als Therapiegerät, dadurch gekennzeichnet, daß zur Abgabe der Schwingungen zwei Magnetköpfe vorgesehen sind und gegebenefalls eine Reizstrom-Handelektrode vorgesehen ist.

20. Vorrichtung nach Anspruch 19, dadurch gekennzeichnet, daß eine weitere Handelektrode vorgesehen ist, die patienteneigene Schwingungen in den Verstärker einbringt.

21. Vorrichtung nach einem der Ansprüche 16 bis 20, dadurch gekennzeichnet, daß die körperspezifischen Spektren in der Kristallgitter eines Festkörpers gespeichert sind.

22. Vorrichtung nach einem der Ansprüche 19 bis 21, dadurch gekennzeichnet, daß die körperspezifischen Spektren die Spektren gesunder Personen sind.

23. Verfahren zur Wiedergabe von körperspezifischen energetischen Informationen in Form von elektromagnetischen Schwingungen, die aus einem analogen Speicher (11, 16, 28) it darin abgespeicherten körperspezifischen Spektren ausgelesen und einen Verstärker zugeleitet werden, dadurch gekennzeichnet, daß das aus dem Verstärker austretende Signal einem oder mehreren Bandpaßfiltern (19 bis 24) zugeführt wird, daß die Ausgangssignale der Bandpaßfilter (19 bis 24) in einer logischen Einheit (25) beliebig kombiniert werden und daß dieses kombinierte Signal der oder den Elektrode(n) (26, 27) zugeleitet wird, wobei die Frequenzbereiche der Bandpaßfilter homöopathischen Potenzen bzw. den folgenden grundlegenden Bereichen - Organe; Bindegewebe, Stütz- und Bewegungsapparat; Vegetativum; bewußte Muskelsteuerung und Triebe (i.e.Selbstverwirklichung); Sinnesorgane und Auswertung (i.e. Lebenserfahrung); abstraktes Denken (i.e.Ego) entsprechen.

24. Verfahren nach Anspruch 23, dadurch gekennzeichnet, daß das aus dem Verstärker austretende Signal wahlweise einem oder mehreren von sechs getrennt ansteuerbaren Bankdpaßfiltern (6) zugeleitet wird, deren Frequenzbereiche den homöopathischen Potenzen D3, D4, D12, D21, D30 und D200 entsprechen.

25. Verfahren nach einem der Ansprüche 23 oder 24, dadurch gekennzeichnet, daß das verstärkte Signal einer der Schuhmann-Welle ähnlichen Trägerwelle überlagert wird.

26. Verfahren nach Anspruch 25, dadurch gekennzeichet, daß die Trägerwelle ein 10Hz Magnetfeld mit 1MHz Impulsen ist.

27. Verfahren nach einem der Ansprüche 23 bis 27, dadurch gekennzeichnet, daß durch Überlagerung des körperspezifischen Spektrums mit patienten eigenen Schwingungen Resonanzzustände erzeugt werden.
